# EUROPÄISCHE PATENTANMELDUNG

(11) **EP 3 964 239 A1**
(43) Veröffentlichungstag der Anmeldung: **09.03.2022**
(21) Anmeldenummer: 20194989.8
(22) Anmeldetag: 08.09.2020
(51) Int. Cl.: A61L 2/08, A61L 2/10, A61L 2/28, A61L 2/00, A61L 2/24, G06T 7/00, G08B 21/24

(54) **VERFAHREN UND VORRICHTUNG ZUR PHOTODYNAMISCHEN DESINFEKTION EINER OBJEKTOBERFLÄCHE**

(71) Anmelder: Photonic Optische Geräte Gesellschaft m.b.H. & Co. KG, 1160 Wien (AT)
(72) Erfinder: ENENGL, Joachim, 1020 Wien (AT); ZOTTER, Stefan, 1190 Wien (AT)
(74) Vertreter: Weiser & Voith Patentanwälte Partnerschaft

(57) **Zusammenfassung**

Die vorliegende Erfindung betrifft ein Verfahren und eine Vorrichtung zur photodynamischen Desinfektion einer Objektoberfläche (3), umfassend:
a) Aufbringen (5) eines farbigen Photosensibilisators (6), der bei Belichtung seine Farbe ändert, auf die Objektoberfläche (3);
b) Erfassen (11) eines ersten Kamerabilds (13) des Objekts (4) mit einer Kamera (12);
c) Bildauswerten (16) des ersten Kamerabilds (13) auf Farblücken (17) mit einem Prozessor (14), und, bei Detektion einer Farblücke (17), Wiederholen der Schritte a) bis c);
d) Aktivieren (18) einer Lichtquelle (19) über eine vorgegebene Dauer;
e) Erfassen (20) eines zweiten Kamerabilds (21) des Objekts (4);
f) Bildauswerten (22) des zweiten Kamerabilds (21) auf lückenhafte Farbänderung, und, bei Detektion einer Farbänderungs-Lücke, Wiederholen der Schritte d) bis f); und
g) Signalisieren (24) der erfolgreichen Desinfektion.

## Beschreibung

Die vorliegende Erfindung betrifft ein Verfahren und eine Vorrichtung zur photodynamischen Desinfektion der Oberfläche eines Objekts.

Bei der photodynamischen Desinfektion von Oberflächen werden im Zusammenspiel von Licht, mit welchem das Objekt belichtet wird, und einem Photosensibilisator (auch "Photosensitizer" genannt), welcher durch Belichtung aktiviert wird, reaktive Sauerstoffspezies ("ROS") erzeugt. ROS sind als unspezifisches Biozid, z.B. aus der Nutzung von Wasserstoffperoxid (H₂O₂), bekannt, d.h. als eine Substanz, welche jede Art von Krankheitserregern, z.B. Pilze (*inklusive deren Sporen*)*,* Bakterien, Parasiten und Viren physikalisch zerstört. ROS sind weder krebserregend noch für den Menschen genotoxisch. Je nach Art des Photosensibilisators wirkt dieser bzw. ein darin enthaltener Wirkstoff bei der photodynamischen Desinfektion häufig als Katalysator, welcher bei Belichtung ROS aus der Umgebungsluft erzeugt; andere Photosensibilisatoren geben Sauerstoff zur Erzeugung von ROS ab. Mit dem vorgesehenen bzw. verwendeten Photosensibilisator und seinem Absorptionsspektrum ist die Wellenlänge der belichtenden Lichtquelle abzustimmen; sie reicht vom Ultraviolett- bis in den Infrarot-Bereich.

Aus der US 2007/0231194 A1 sind Verfahren und Systeme zur Sterilisation und Oberflächen-Desinfektion von Objekten bekannt. Dabei wird in einigen Varianten ein durch Belichtung aktiviertes Desinfektionsmittel auf das Objekt, z.B. ein medizinisches Instrument oder die Hände einer Person, aufgetragen und belichtet. Um die ausreichende Belichtung nachprüfbar zu machen, wird optional das Objekt zusätzlich mit einem lichtempfindlichen Indikator-Etikett versehen, welches bei der Belichtung seine Farbe ändert, sodass an der Farbänderung die ausreichende Belichtung erkennbar ist. In einer anderen Variante werden lichtempfindliche Indikator-Handschuhe verwendet bzw. wird lichtempfindliche Indikator-Farbe auf das Objekt aufgesprüht. All diesen Varianten ist gemeinsam, dass zwar die Belichtung - zumindest an der Stelle des Etiketts - geprüft wird, dadurch jedoch noch nicht auf die sichere Desinfektion der Objektoberfläche geschlossen werden kann.

Die Erfindung setzt sich zum Ziel, ein Verfahren und eine Vorrichtung zur photodynamischen Desinfektion einer Objektoberfläche zu schaffen, welche rasch, einfach und sicher einsetzbar sind.

Das Ziel wird gemäß einem ersten Aspekt der Erfindung mit einem Verfahren zur photodynamischen Desinfektion der Objektoberfläche erreicht, welches die folgenden Schritte umfasst:
a) Aufbringen eines farbigen Photosensibilisators, welcher bei Belichtung seine Farbe ändert, auf die Objektoberfläche;
b) Erfassen eines ersten Kamerabilds des Objekts mit zumindest einer Kamera;
c) Computer-gestütztes Bildauswerten des ersten Kamerabilds auf Farblücken des auf die Objektoberfläche aufgebrachten Photosensibilisators, und, bei Detektion einer Farblücke, Wiederholen der Schritte a) bis c);
d) Aktivieren zumindest einer auf das Objekt gerichteten Lichtquelle über eine vorgegebene Dauer;
e) Erfassen eines zweiten Kamerabilds des Objekts mit der zumindest einen Kamera;
f) Computer-gestütztes Bildauswerten des zweiten Kamerabilds auf lückenhafte Farbänderung des auf die Objektoberfläche aufgebrachten Photosensibilisators, und, bei Detektion einer Farbänderungs-Lücke, Wiederholen der Schritte d) bis f); und
g) Signalisieren der erfolgreichen Desinfektion.

Da in diesem Verfahren zunächst das lückenlose Aufbringen des Photosensibilisators auf die Objektoberfläche und nach dem Belichten zusätzlich dessen lückenlose Farbänderung und folglich die vollflächige Desinfektion der Objektoberfläche geprüft werden, ist die Desinfektion der Objektoberfläche nachvollziehbar zuverlässig und somit besonders sicher. "Farbe" bezeichnet hier die entsprechende optische Eigenschaft des Photosensibilisators nicht nur im sichtbaren Licht-Wellenlängenbereich, sondern umfasst eine vergleichbare optische Eigenschaft im Ultraviolett- bzw. Infrarot-Bereich. Da das Objekt während des Verfahrens nicht bewegt werden muss, ist das Verfahren auch sehr einfach und, je nach erforderlicher Dauer der Belichtung des Photosensibilisators, auch rasch durchzuführen; die typische Belichtungsdauer beträgt etwa 5 Sekunden oder weniger und ist somit wesentlich kürzer als z.B. bei einer herkömmlichen alkoholischen Desinfektion. Die Einfachheit und rasche Durchführbarkeit des Verfahrens erhöhen seine Nutzbarkeit und die Wirksamkeit der Desinfektion. Dabei ist das Verfahren nicht auf bestimmte Objekte oder Objekteigenschaften eingeschränkt und insbesondere zur Desinfektion von Händen geeignet.

In einer vorteilhaften Ausführungsform wird der Photosensibilisator separat auf die Objektoberfläche aufgebracht. Diese Ausführungsform umfasst den zwischen den Schritten a) und b) ausgeführten Schritt des Einführens des Objekts in eine Belichtungskammer, welche mit der zumindest einen Kamera und der zumindest einen Lichtquelle bestückt ist. Eine dazu alternative Ausführungsform umfasst den vor Schritt a) ausgeführten Schritt des Einführens des Objekts in die Belichtungskammer, welche mit der zumindest einen Kamera, der zumindest einen Lichtquelle und zumindest einer Düse bestückt ist, mit welcher Düse in Schritt a) der Photosensibilisator auf die Objektoberfläche aufgebracht wird. Das Verfahrens ist in dieser Ausführungsform besonders einfach und rasch durchführbar.

Eine günstige Variante der beiden vorgenannten Ausführungsformen umfasst die unmittelbar im Anschluss an den Schritt des Einführens ausgeführten Schritte:
i) Erfassen eines weiteren Kamerabilds des Objekts mit der zumindest einen Kamera; und
ii) Computer-gestütztes Bildauswerten des weiteren Kamerabilds auf Übereinstimmung mit einer vorgegebenen Form und/oder Lage des Objekts, und, bei Detektion mangelnder Übereinstimmung, Signalisieren einer Abweichung und Wiederholen der Schritte i) und ii).

Dadurch wird sichergestellt, dass die Objektoberfläche wie vorgegeben im Kamerabild erfasst wird, sodass das Verfahren auch auf Objekte komplexer Form flexibel und sicher anwendbar ist. Beispielsweise kann überprüft werden, ob beim Desinfizieren einer Hand die Hand geöffnet und die Finger ausreichend gespreizt sind bzw. ob das Objekt die vorgegebene Lage, d.h. Position und Orientierung, im Inneren der Belichtungskammer hat. Es versteht sich, dass diese Schritte i) und ii) alternativ oder ergänzend unmittelbar vor den Schritten b) bzw. e) des Erfassens der ersten bzw. zweiten Kamerabilds ausgeführt werden können. Dazu alternativ könnte der Schritt ii) jeweils zwischen den Schritten b) und c) und/oder e) und f) durchgeführt werden und sich dabei jeweils - anstelle des weiteren Kamerabilds aus Schritt i) - auf das erste Kamerabild aus Schritt b) bzw. das zweite Kamerabild aus Schritt e) stützen.

Als Photosensibilisator mit den gewünschten Eigenschaften kommen verschiedene Stoffe bzw. Stoffgemische infrage, insbesondere Wirkstoffe, welche unter Belichtung sowohl ROS erzeugen als auch ihre Farbe ändern, bzw. Stoffgemische mit zumindest einem solchen Wirkstoff oder Stoffgemische mit zumindest einem unter Belichtung ROS-erzeugenden Wirkstoff und zumindest einem unter Belichtung seine Farbe ändernden Farbstoff. Als besonders wirksam und zuverlässig hat sich erwiesen, wenn der Photosensibilisator zumindest einen Wirkstoff aus der Gruppe Chlorophyll, Curcumin und 5-Aminolävulinsäure-induziertes Protoporphyrin IX enthält.

In einem zweiten Aspekt schafft die Erfindung eine Vorrichtung zur photodynamischen Desinfektion der Oberfläche eines Objekts, umfassend ein Gehäuse mit einer Belichtungskammer, welche mit zumindest einer Lichtquelle zum Belichten des Objekts bestückt ist, welche Vorrichtung sich dadurch auszeichnet, dass die Belichtungskammer ferner mit zumindest einer Kamera zum Erfassen von Kamerabildern des Objekts bestückt ist und die Vorrichtung ferner einen Prozessor und eine Signalisierungseinrichtung umfasst, wobei der Prozessor dazu ausgebildet ist, gemäß dem Verfahren der vorgenannten Art die von der Kamera erfassten Kamerabilder bildauszuwerten und die Lichtquelle und die Signalisierungseinrichtung zu steuern. Bezüglich der Vorteile und weiterer Ausführungsformen der Vorrichtung wird ergänzend auf die vorangegangenen Ausführungen zum Verfahren verwiesen.

In einer günstigen Variante hat die zumindest eine Kamera eine öffenbare Schutzabdeckung. Eine solche Schutzabdeckung bietet einen guten Schutz vor Verschmutzung der Kamera z.B. durch den Photosensibilisator. Es versteht sich, dass zusätzlich oder alternativ die zumindest eine Lichtquelle mit einer vergleichbaren öffenbaren Schutzabdeckung versehen sein kann.

Vorteilhaft ist, wenn die Belichtungskammer ferner eine von der zumindest einen Kamera erfasste Spiegelfläche umfasst. Dies ermöglicht es der zumindest einen Kamera, aus ihrer Kameraposition nicht direkt sichtbare Bereiche der Objektoberfläche dennoch im Kamerabild zu erfassen. Durch eine oder mehrere solcher Spiegelflächen kann die Anzahl der verwendeten Kameras in der Belichtungskammer reduziert werden, was das Verfahren und die Vorrichtung vereinfacht. Als Spiegelflächen kommen einerseits spiegelnde Innenwandflächen der Belichtungskammer selbst oder separate, in der Belichtungskammer angeordnete, z.B. konkave Spiegel infrage.

Alternativ oder ergänzend dazu ist es günstig, wenn die Belichtungskammer mit mehreren Kameras bestückt ist, welche in der Belichtungskammer verteilt sind und gemeinsam die Kamerabilder erfassen. Dies ermöglicht die Erfassung der gesamten Objektoberfläche durch die Kameras. Jedes Kamerabild wird somit von allen Kameras gemeinsam erfasst, z.B. durch einfaches zur-Verfügung-Stellen oder Aneinanderreihen von Bildern aus verschiedenen Blickwinkeln; ein Zusammenheften des Objekts ("stitching") aus mehreren Bildern ist möglich, jedoch nicht erforderlich.

Vorteilhaft ist ferner, wenn die Belichtungskammer mit mehreren Lichtquellen bestückt ist, welche in der Belichtungskammer verteilt und vom Prozessor gemeinsam gesteuert sind. Dies ermöglicht ein gleichmäßiges und gleichzeitiges Belichten der gesamten Objektoberfläche und somit die rasche und sichere Durchführung des Verfahrens.

In einer bevorzugen Ausführungsform umfasst die Vorrichtung ferner einen Behälter für einen Photosensibilisator und ist die Belichtungskammer ferner mit zumindest einer aus dem Behälter gespeisten und vom Prozessor gesteuerten Düse zum Aufbringen des Photosensibilisators auf die Objektoberfläche bestückt. Dies ermöglicht eine vollautomatische Ausführung des Verfahrens, wodurch seine Anwendung besonders einfach und sicher ist. Günstig ist dabei, wenn die Belichtungskammer mit mehreren Düsen bestückt ist, welche in der Belichtungskammer verteilt und vom Prozessor gemeinsam gesteuert sind. Auf diese Weise wird die Gleichmäßigkeit des Aufbringens des Photosensibilisators auf die Objektoberfläche gefördert und das Aufbringen beschleunigt.

Die Erfindung wird nachfolgend anhand von in den beigeschlossenen Zeichnungen dargestellten Ausführungsbeispielen näher erläutert. In den Zeichnungen zeigen:
Fig. 1 ein Verfahren gemäß der Erfindung zur photodynamischen Desinfektion der Oberfläche eines Objekts in einem Flussdiagramm; und
Fig. 2 eine Variante einer Vorrichtung zum Ausführen des Verfahrens von Fig. 1 in einer Perspektivansicht von schräg oben.

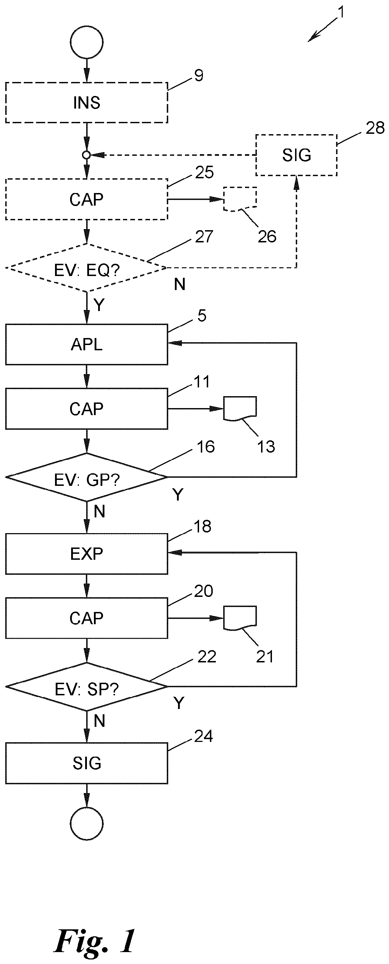

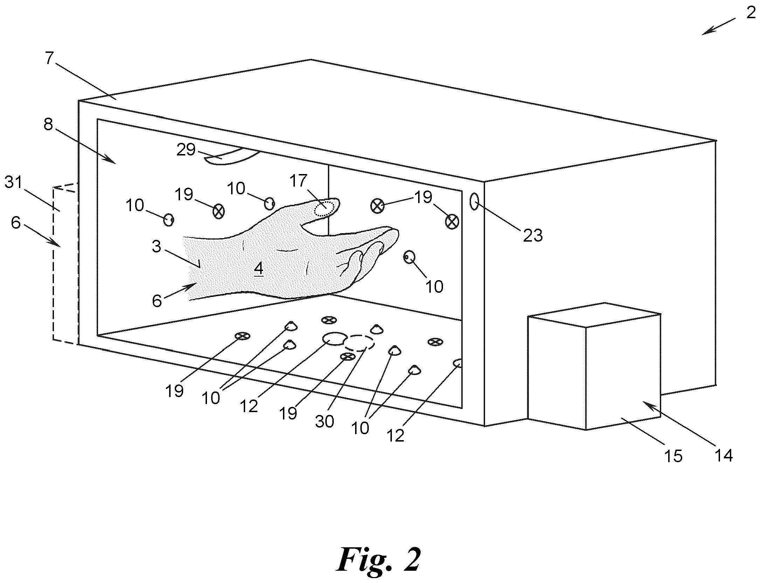

Die Fig. 1 und 2 zeigen Beispiele für ein Verfahren (Fig. 1) und eine Vorrichtung (Fig. 2) zur photodynamischen Desinfektion der Oberfläche 3 eines Objekts 4. Das Objekt 4 kann unterschiedlicher Art sein und verschiedene Eigenschaften haben; im Beispiel der Fig. 2 ist das Objekt 4 eine menschliche Hand, kann jedoch ein völlig anderes Objekt 4 sein.

Eine photodynamische Desinfektion dient zur Inaktivierung von Keimen - z.B. Pilzen (inklusive deren Sporen), Bakterien, Parasiten und Viren - auf Objektoberflächen 3 mithilfe von Licht in Kombination mit einem Photosensibilisator (auch "Photosensitizer" genannt). Im Zusammenspiel von Licht und Photosensibilisator werden aus Sauerstoff meist aus der Umgebungsluft, seltener aus dem Photosensibilisator selbst, reaktive Sauerstoffspezies (ROS) erzeugt. Die ROS zerstören die Krankheitserreger physikalisch, sind jedoch für den Menschen weder genotoxisch noch krebserregend.

In einem ersten Schritt 5 des Verfahrens 1 wird zur photodynamischen Desinfektion ein Photosensibilisator 6 (Fig. 2) auf die Oberfläche 3 des Objekts 4 aufgebracht. Der in dem Verfahren 1 und in der Vorrichtung 2 verwendete Photosensibilisator 6 ist farbig und ändert seine Farbe bei Belichtung. Zu diesem Zweck kann der eigentliche lichtaktive (d.h. ROS-erzeugende) Wirkstoff des Photosensibilisators 6 selbst farbig sein und bei Belichtung seine Farbe ändern (z.B. auch bleichen). Beispielsweise kann der Photosensibilisator 6 zumindest einen Wirkstoff aus der Gruppe Chlorophyll (z.B. Chlorophyll a oder Chlorophyll b), Curcumin und 5-Aminolävulinsäure-induziertes Protoporphyrin IX enthalten. Alternativ oder ergänzend enthält der Photosensibilisator - neben dem ROS-erzeugenden Wirkstoff - einen zusätzlichen, bei Belichtung seine Farbe ändernden Farbstoff.

In diesem Zusammenhang sei angemerkt, dass die Begriffe "Farbe" bzw. "farbig" in Bezug auf den genannten Photosensibilisator die entsprechende optische Eigenschaft nicht nur im sichtbaren Licht-Wellenlängenbereich bezeichnen, sondern eine vergleichbare optische Eigenschaft im Ultraviolett- bzw. Infrarot-Bereich mitumfassen.

Der Schritt des Aufbringens 5 des Photosensibilisators 6 auf die Objektoberfläche 3 erfolgt dabei in einer Ausführungsform separat, d.h. unabhängig von der Vorrichtung 2, z.B. durch Einreiben. Danach wird das Objekt 4 mit aufgebrachtem Photosensibilisator 6 in eine in einem Gehäuse 7 der Vorrichtung 2 angeordnete Belichtungskammer 8 zur Ausführung der weiter unten beschriebenen nachfolgenden Verfahrensschritte eingeführt. Alternativ dazu wird das Objekt 4 bereits vor dem Aufbringen 5 des Photosensibilisators 6 auf die Objektoberfläche 3 in die Belichtungskammer 8 eingeführt (Schritt 9 in Fig. 1) und ist die Belichtungskammer 8 mit zumindest einer Düse 10 bestückt, mit welcher der Photosensibilisator 6 in Schritt 5 auf die Objektoberfläche 3 aufgebracht (z.B. aufgesprüht) wird.

In einem folgenden Schritt 11 wird mit zumindest einer Kamera 12, mit welcher die Belichtungskammer 8 bestückt ist, ein erstes Kamerabild 13 des Objekts 4 erfasst. Die zumindest eine Kamera 12 ist eine Foto- oder Videokamera. Entsprechend erfasst die zumindest eine Kamera 12 das erste Kamerabild 13 von dem in die Belichtungskammer 8 eingeführten Objekt 4 z.B. regelmäßig oder auf Veranlassung durch einen Prozessor 14 der Vorrichtung 2, welcher im Beispiel der Fig. 2 in einem Steuerungsmodul 15 der Vorrichtung 2 angeordnet ist (alternativ könnte der Prozessor 14 völlig separat vom oder direkt im Gehäuse 7 der Vorrichtung 2 angeordnet sein); oder es entnimmt z.B. der Prozessor 14 das von der zumindest einen Kamera 12 erfasste erste Kamerabild 13 bei Bedarf einem Videosignal der Kamera 12.

In einem weiteren Schritt 16 wird das erste Kamerabild 13 mithilfe des Prozessors 14, d.h. Computer-gestützt, bildausgewertet. Dabei ermittelt der Prozessor 14, ob der auf die Objektoberfläche 3 aufgebrachte Photosensibilisator 6 eine oder mehrere Farblücken 17 aufweist. Wird zumindest eine Farblücke 17 detektiert (Zweig "Y" beim Bildauswerten 16 in Fig. 1), werden die Schritte des Aufbringens 5, Erfassens 11 des ersten Kamerabilds 13 und des Computer-gestützten Bildauswertens 16 wiederholt.

Wird keine Farblücke 17 detektiert (Zweig "N" beim Bildauswerten 16 in Fig. 1), aktiviert der Prozessor 14 in einem folgenden Schritt 18 zur Belichtung des auf der Objektoberfläche 3 aufgebrachten Photosensibilisators 6 zumindest eine darauf gerichtete Lichtquelle 19, mit welcher die Belichtungskammer 8 ebenfalls bestückt ist, über eine vorgegebene Dauer. Die vorgegebene Dauer hängt in dem Fachmann bekannter Weise von der Lichtquelle 19 und dem verwendeten Photosensibilisator 6, d.h. insbesondere einerseits von der Lichtwellenlänge und der Lichtstärke der Lichtquelle 19 und andererseits von einer Konzentration des ROS-erzeugenden Wirkstoffs bzw. der Art und dem Absorptionsspektrum des Photosensibilisators 6, ab, wobei Lichtwellenlänge und Absorptionsspektrum im sichtbaren, im Ultraviolett- oder im Infrarot-Bereich liegen können; im Allgemeinen beträgt die Dauer wenige - z.B. fünf - Sekunden.

In weiterer Folge wird in Schritt 20 mit der zumindest einen Kamera 12 ein zweites Kamerabild 21 des Objekts 4 erfasst und darauf in Schritt 22 mithilfe des Prozessors 14 das zweite Kamerabild 21 bildausgewertet. Dabei ermittelt der Prozessor 14, ob der auf die Objektoberfläche 3 aufgebrachte Photosensibilisator 6 in Schritt 18 ausreichend belichtet wurde und somit an der gesamten Objektoberfläche 3 seine Farbe geändert hat, in welchem Fall die photodynamische Desinfektion der Objektoberfläche 3 vollflächig erfolgreich war. War die Farbänderung lückenhaft (Zweig "Y" beim Bildauswerten 22 in Fig. 1), sodass der Prozessor 14 beim Bildauswerten 22 eine oder mehrere Farbänderungs-Lücken (in Fig. 2 nicht gezeigt) detektiert, dann werden die Schritte des Aktivierens 18 der Lichtquelle 19, des Erfassens 20 des zweiten Kamerabilds 21 und des Bildauswertens 22 des zweiten Kamerabilds 22 wiederholt. Ergibt das Bildauswerten 22 hingegen eine lückenlose Farbänderung des Photosensibilisators 6 auf der gesamten Objektoberfläche 3 (Zweig "N" beim Bildauswerten 22 in Fig. 1), steuert der Prozessor 14 eine Signalisierungseinrichtung 23 der Vorrichtung 2, z.B. eine Leuchte, einen Lautsprecher oder ein Display an, sodass diese in einem abschließenden Schritt 24 die erfolgreiche Desinfektion der Objektoberfläche 3 signalisiert.

In einer optionalen Ausführungsform werden folgende zusätzliche Schritte ausgeführt, u.zw. unmittelbar im Anschluss an das Einführen 9 des Objekts 4 in die Belichtungskammer 8, d.h., wenn das Aufbringen 5 des Photosensibilisators 6 auf die Objektoberfläche 3 außerhalb der Belichtungskammer 8 erfolgt ist, unmittelbar vor dem Erfassen 11 des ersten Kamerabilds 13, oder, wenn das Aufbringen 5 des Photosensibilisators 6 mithilfe der zumindest einen Düse 10 nach dem Einführen 9 und somit im Inneren der Belichtungskammer 8 durchgeführt wird, unmittelbar vor dem Aufbringen 5. Zunächst wird in Schritt 25 ein weiteres Kamerabild 26 des Objekts 4 mit der zumindest einen Kamera 12 erfasst und im folgenden Schritt 27 das weitere Kamerabild 26 Computer-gestützt bildausgewertet; dabei prüft der Prozessor 14, ob das Objekt 4 in dem erfassten weiteren Kamerabild 26 mit einer vorgegebenen Form und/oder einer vorgegebenen Lage des Objekts übereinstimmt. Detektiert der Prozessor 14 dabei eine mangelnde Übereinstimmung (Zweig "N" beim Bildauswerten 27 in Fig. 1), stimmt somit das weitere Kamerabild 26 des Objekts 4 nicht mit der vorgegebenen Form (z.B. einer offenen Handhaltung mit gespreizten Fingern) und/oder der vorgegebenen Lage (d.h. Position und Orientierung im Inneren der Belichtungskammer 8) überein, steuert der Prozessor 14 die Signalisierungseinrichtung 23 zum Signalisieren (Schritt 28) einer Abweichung an, sodass vor dem jeweils folgenden Schritt 5 oder 11 die vorgegebene Form oder Lage hergestellt bzw. eingenommen werden kann; die Schritte des Erfassens 25 des weiteren Kamerabilds 26 und des Bildauswertens 27 des weiteren Kamerabilds 26 - erforderlichenfalls inklusive des Signalisierens 28 der Abweichung - werden darauf wiederholt. Detektiert der Prozessor 14 andererseits beim Bildauswerten 27 des weiteren Kamerabilds 26 die Übereinstimmung (Zweig "Y" beim Bildauswerten 27 in Fig. 1), wird mit dem jeweils folgenden Aufbringen 5 oder Erfassen 11 fortgesetzt.

Es versteht sich, dass die vorgenannten Schritte Erfassen 25 und Bildauswerten 27 des weiteren Kamerabilds 26 sowie - erforderlichenfalls - Signalisieren 28 einer Abweichung und deren Wiederholen optional zusätzlich oder alternativ vor den Schritten 11 und 20 des Erfassens des ersten bzw. zweiten Kamerabilds 13, 21 durchgeführt werden können. Ferner kann optional bloß der Schritt des Bildauswertens 27 auf Grundlage des ersten bzw. zweiten Kamerabilds 13, 21 deren Übereinstimmung mit der vorgegebenen Form und/oder der vorgegebenen Lage jeweils vor den zugehörigen Schritten des Bildauswertens 16, 22 des ersten bzw. des zweiten Kamerabilds 13, 21 durchgeführt werden; erforderlichenfalls wird auch hier der Schritt des Signalisierens 28 einer Abweichung und das Wiederholen sinngemäß stattfinden.

Fig. 2 zeigt eine Variante der Vorrichtung 2, bei welcher die Belichtungskammer 8 in dem Gehäuse 7 mit mehreren Lichtquellen 19 bestückt ist, welche in der Belichtungskammer 8 verteilt sind. Die Lichtquellen 19 werden vom Prozessor 14 gemeinsam gesteuert, d.h. gleichzeitig aktiviert und nach Ablauf der vorgegeben Dauer deaktiviert. Es versteht sich, dass alternativ zumindest einige der Lichtquellen 19 vom Prozessor auch separat gesteuert werden können, z.B. in Abhängigkeit von dem in die Belichtungskammer 8 eingeführten Objekt 4.

Die Belichtungskammer 8 ist im Beispiel der Fig. 2 ferner mit mehreren (in der Perspektivansicht von Fig. 2 sichtbar: zwei) Kameras 12 bestückt, welche ebenfalls in der Belichtungskammer 8 verteilt sind und die Kamerabilder 13, 21, 26 jeweils gemeinsam erfassen, d.h. jedes Kamerabild 13, 21, 26 enthält die Bilder aller Kameras 12, z.B. durch Aneinanderreihen mit oder ohne Aneinanderheften ("stitching"). Alternativ wertet der Prozessor 14 die von den mehreren Kameras 12 erfassten Bilder als gemeinsam erfasstes Kamerabild 13, 21, 26 einzeln aus.

Ferner umfasst die Belichtungskammer 8 optional eine von der zumindest einen Kamera 12 erfasste Spiegelfläche (hier einen Konvexspiegel) 29, über welchen die Kamera 12 einen weiteren Blickwinkel auf das in die Belichtungskammer 8 eingeführte Objekt 4 und seine Oberfläche 3 erhält. Zumindest eine der beiden in Fig. 2 dargestellten Kameras 12 hat eine optionale Schutzabdeckung 30, welche zum Schutz der Kamera 12 über diese geschoben, geschwenkt, geklappt etc. oder nach Art einer Blende über dieser geschlossen und vor dem Erfassen der Kamerabilder 13, 21, 26 geöffnet wird, d.h. die Kamera 12 freigibt.

Optional umfasst die Vorrichtung 2 ferner einen Behälter 31 für den Photosensibilisator 6, welcher Behälter 31 die zumindest eine Düse 10 speist. Die zumindest eine Düse 10 wird zum Aufbringen 5 des Photosensibilisators 6 auf die Objektoberfläche 3 von dem Prozessor 14 gesteuert. In dem dargestellten Beispiel ist die Belichtungskammer 8 mit mehreren Düsen 10 bestückt, welche in der Belichtungskammer 8 verteilt und vom Prozessor 14 gemeinsam - oder alternativ: einzeln oder in Gruppen - gesteuert sind.

Die Formulierung "in der Belichtungskammer 8 verteilt" bedeutet in diesem Zusammenhang, dass die mehreren Düsen 10, Kameras 12 und/oder Lichtquellen 19 voneinander beabstandet und dabei insbesondere an der Wandung der Belichtungskammer 8 angeordnet oder - wie in dem dargestellten Beispiel - in die Wandung integriert sind, u.zw. optional auch an verschiedenen Seiten der Wandung, z.B. unten, oben, links, rechts und/oder hinten in der Belichtungskammer 8. Ferner sei angemerkt, dass die Belichtungskammer 8 nicht zwingend quaderförmig, sondern eine beliebig geformte, von außen zugängliche Kammer oder Vertiefung in dem Gehäuse 7 ist.

Die Erfindung ist nicht auf die dargestellten Ausführungsformen beschränkt, sondern umfasst alle Varianten, Modifikationen und deren Kombinationen, die in den Rahmen der angeschlossenen Ansprüche fallen.

## Patentansprüche

1. Verfahren zur photodynamischen Desinfektion der Oberfläche (3) eines Objekts (4), umfassend die Schritte:
a) Aufbringen (5) eines farbigen Photosensibilisators (6), welcher bei Belichtung seine Farbe ändert, auf die Objektoberfläche (3);
b) Erfassen (11) eines ersten Kamerabilds (13) des Objekts (4) mit zumindest einer Kamera (12);
c) Computer-gestütztes Bildauswerten (16) des ersten Kamerabilds (13) auf Farblücken (17) des auf die Objektoberfläche (3) aufgebrachten Photosensibilisators (6), und, bei Detektion einer Farblücke (17), Wiederholen der Schritte a) bis c);
d) Aktivieren (18) zumindest einer auf das Objekt (4) gerichteten Lichtquelle (19) über eine vorgegebene Dauer;
e) Erfassen (20) eines zweiten Kamerabilds (21) des Objekts (4) mit der zumindest einen Kamera (12);
f) Computer-gestütztes Bildauswerten (22) des zweiten Kamerabilds (21) auf lückenhafte Farbänderung des auf die Objektoberfläche (3) aufgebrachten Photosensibilisators (6), und, bei Detektion einer Farbänderungs-Lücke, Wiederholen der Schritte d) bis f); und
g) Signalisieren (24) der erfolgreichen Desinfektion.

2. Verfahren nach Anspruch 1, **gekennzeichnet durch** den zwischen den Schritten a) und b) ausgeführten Schritt des Einführens (9) des Objekts (4) in eine Belichtungskammer (8), welche mit der zumindest einen Kamera (12) und der zumindest einen Lichtquelle (19) bestückt ist.

3. Verfahren nach Anspruch 1, **gekennzeichnet durch** den vor Schritt a) ausgeführten Schritt des Einführens (9) des Objekts (4) in eine Belichtungskammer (8), welche mit der zumindest einen Kamera (12), der zumindest einen Lichtquelle (19) und zumindest einer Düse (10) bestückt ist, mit welcher in Schritt a) der Photosensibilisator (6) auf die Objektoberfläche (3) aufgebracht wird.

4. Verfahren nach Anspruch 2 oder 3, **gekennzeichnet durch** die unmittelbar im Anschluss an den Schritt des Einführens (9) ausgeführten Schritte:
i) Erfassen (25) eines weiteren Kamerabilds (26) des Objekts (4) mit der zumindest einen Kamera (12); und
ii) Computer-gestütztes Bildauswerten (27) des weiteren Kamerabilds (26) auf Übereinstimmung mit einer vorgegebenen Form und/oder Lage des Objekts (4), und, bei Detektion mangelnder Übereinstimmung, Signalisieren (28) einer Abweichung und Wiederholen der Schritte i) und ii).

5. Verfahren nach einem der Ansprüche 1 bis 4, **dadurch gekennzeichnet, dass** der Photosensibilisator (6) zumindest einen Wirkstoff aus der Gruppe Chlorophyll, Curcumin und 5-Aminolävulinsäure-induziertes Protoporphyrin IX enthält.

6. Vorrichtung zur photodynamischen Desinfektion der Oberfläche (3) eines Objekts (4), umfassend ein Gehäuse (7) mit einer Belichtungskammer (8), welche mit zumindest einer Lichtquelle (19) zum Belichten des Objekts (4) bestückt ist, **dadurch gekennzeichnet, dass** die Belichtungskammer (8) ferner mit zumindest einer Kamera (12) zum Erfassen von Kamerabildern (13, 21, 26) des Objekts (4) bestückt ist und die Vorrichtung (2) ferner einen Prozessor (14) und eine Signalisierungseinrichtung (23) umfasst, wobei der Prozessor (14) dazu ausgebildet ist, gemäß dem Verfahren (1) nach einem der Ansprüche 1 bis 5 die von der Kamera (12) erfassten Kamerabilder (13, 21, 26) bildauszuwerten und die Lichtquelle (19) und die Signalisierungseinrichtung (23) zu steuern.

7. Vorrichtung nach Anspruch 6, **dadurch gekennzeichnet, dass** die zumindest eine Kamera (12) eine öffenbare Schutzabdeckung (30) hat.

8. Vorrichtung nach Anspruch 6 oder 7, **dadurch gekennzeichnet, dass** die Belichtungskammer (8) ferner eine von der zumindest einen Kamera (12) erfasste Spiegelfläche (29) umfasst.

9. Vorrichtung nach einem der Ansprüche 6 bis 8, **dadurch gekennzeichnet, dass** die Belichtungskammer (8) mit mehreren Kameras (12) bestückt ist, welche in der Belichtungskammer (8) verteilt sind und gemeinsam die Kamerabilder (13, 21, 26) erfassen.

10. Vorrichtung nach einem der Ansprüche 6 bis 9, **dadurch gekennzeichnet, dass** die Belichtungskammer (8) mit mehreren Lichtquellen (19) bestückt ist, welche in der Belichtungskammer (8) verteilt und vom Prozessor (14) gemeinsam gesteuert sind.

11. Vorrichtung nach einem der Ansprüche 6 bis 10, **dadurch gekennzeichnet, dass** die Vorrichtung (2) ferner einen Behälter (31) für einen Photosensibilisator (6) umfasst, und dass die Belichtungskammer (8) ferner mit zumindest einer aus dem Behälter (31) gespeisten und vom Prozessor (14) gesteuerten Düse (10) zum Aufbringen des Photosensibilisators (6) auf die Objektoberfläche (3) bestückt ist.

12. Vorrichtung nach Anspruch 11, **dadurch gekennzeichnet, dass** die Belichtungskammer (8) mit mehreren Düsen (10) bestückt ist, welche in der Belichtungskammer (8) verteilt und vom Prozessor (14) gemeinsam gesteuert sind.
